# EUROPEAN PATENT SPECIFICATION

(11) **EP 2 995 335 B1**
(45) Date of publication and mention of the grant of the patent: **12.10.2022**
(21) Application number: 15183196.3
(22) Date of filing: 31.08.2015
(51) Int. Cl.: A61M 25/00, A61M 25/10

(54) **DRUG COATED BALLOON CATHETER AND METHOD OF MANUFACTURE THEREOF**
MEDIKAMENTENBESCHICHTETER BALLONKATHETER UND VERFAHREN ZUR HERSTELLUNG DAVON
CATHÉTER À BALLONNET REVÊTU DE MÉDICAMENT ET SON PROCÉDÉ DE FABRICATION

(30) Priority: 11.09.2014 IT PD20140233
(43) Date of publication of application: 16.03.2016
(73) Proprietor: Pine Medical Limited, Hong Kong (HK)
(72) Inventor: SCHAFFNER, Silvio, Hong Kong (HK)
(74) Representative: Mitola, Marco

(56) References cited:
- US-A1- 2006 004 323
- US-A1- 2009 254 063
- US-A1- 2012 143 054
- US-A1- 2013 237 950

## Description

The present invention relates to a drug coated balloon catheter and the method of manufacture thereof.

In particular, it is known in the art to realize drug coated balloon (DCB) catheters comprising a shaft, extending from a tip (distal) to a connector (proximal), and a balloon which is expandable and which is covered or soaked with drug, in order to elute its drug directly on the target lesion.

Known Drug Coated Balloon Catheters (DCB catheters) are not lubricant coated. This leads to the fact that the DCB has higher friction in the interior wall of the target vessel or the interior wall of an introducer, sheath, guiding catheter or any other accessory used for intervention; and to the exterior wall of the guide wire used for intervention; therefore the catheter has problems to reach, or even cannot reach, the target lesion.

Sometimes the DCB catheter reaches the target lesion but, when moving into the vessel, it damages it because of said high friction: this damage can, for example, occur in correspondence of reductions of the lumen of the vessel or in correspondence of bifurcations of the vessel itself.

Moreover, the DCB catheters may encounter high friction and/or jamming between the internal wall of the shaft, defining the guidewire lumen, and the guidewire itself: this friction/sticking increases the problems of the balloon to reach the target lesion.

The problem of high friction between the guidewire lumen and the guidewire is increased in DCB catheters which have catheter shafts with coaxial or dual-lumen construction: one separate lumen is used so as to inflate the balloon. In fact, in such devices the guidewire lumen is reduced in diameter and then there is higher risk of blocking of the guidewire against the internal wall of the guidewire lumen.

Moreover, DCB catheters encounter higher friction than other types of balloon catheters due to the drug coating on the surface of the balloon; therefore the risk of jamming or grasping within blood vessel is really increased when using a DCB catheter with respect to other types of balloon catheters.

US 2012/0143054 A1 describes a coated balloon catheter having a multi-lumen structure.

US 2013/0237950 A1 describes a length and diameter adjustable balloon catheter with a multi-lumen structure.

Therefore, it is not known in the state of the art a solution of DCB catheter which can easily reach the target lesion, without jamming against the internal wall of the vessel or against the guidewire.

The purpose of the present invention is that of providing

a catheter which overcomes the drawbacks mentioned with reference to the prior art; in other words a DCB catheter which can easily reach the target lesion without jamming and without damaging the internal wall of the cannulated vessel.

Such aim is reached by a catheter according to claim 1 and by a method according to claim 9.

Other embodiments of the catheter according to the invention are described in the subsequent claims.

Further characteristics and advantages of the present invention will be more clearly comprehensible from the description given below of its preferred and non-limiting embodiments, wherein:

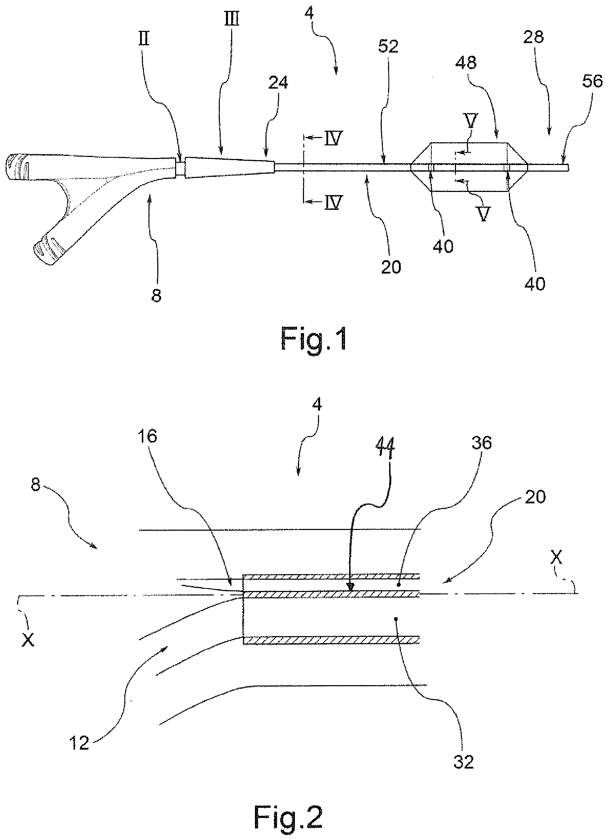

- figure 1 shows a perspective view of a drug coated balloon (DCB) catheter according to an embodiment of the present invention;
- figure 2 shows a longitudinal section view of particular II of the catheter of figure 1;

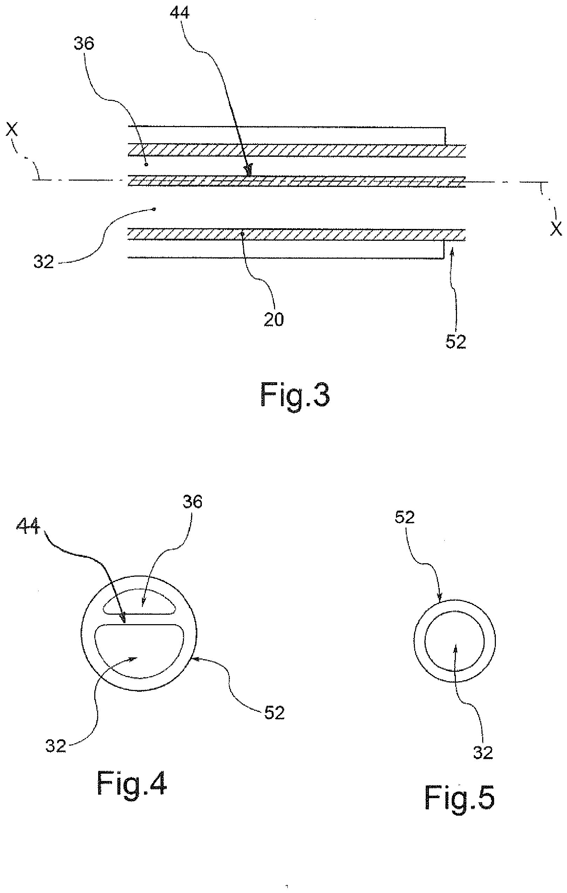

- figure 3 shows a longitudinal section view of particular III of the catheter of figure 1;
- figure 4 shows a section view of the catheter of figure 1, taken along section line IV-IV shown in figure 1;
- figure 5 shows a section view of the catheter of figure 1, taken along section line V-V shown in figure 1.

The elements or parts of elements common to the embodiments described below will be indicated using the same reference numerals.

With reference to the aforementioned figures, reference numeral 4 globally denotes a catheter, in particular, a drug coated balloon (DCB) catheter for releasing drug on a target lesion inside a blood vessel.

The catheter 4 comprises a connector 8 comprising a guide wire channel 12 and an inflation channel 16.

The guide wire channel 12 is useful for housing a guide wire for the guide and the insertion of the catheter 4 in a predetermined vessel.

Moreover, the inflation channel 16 is useful in order to send a pressure fluid to the distal end of the shaft of the catheter. For example the pressure fluid may be gas or a liquid. Moreover, the inflation channel may be used as a perfusion channel, for example, of a contrast liquid.

The catheter 4 comprises a shaft 20 extending from a proximal end 24 to a distal end 28 along an axial direction X-X.

Moreover, the shaft 20 have a guide wire lumen 32 and an inflation lumen 36.

In particular, the catheter according to the present invention has at least a dual-lumen construction, comprising at least two lumens comprising a guidewire lumen 32 and an inflation lumen 36.

Preferably, said guidewire lumen 32 and inflation lumen 36 are not coaxial with each other. Said guidewire lumen 32 and inflation lumen 36 are separated by an internal sept 44. Said internal sept 44 delimits, at least partly, a wall of each of the separated lumens 32,36.

Anyway, the catheter 4 may have further lumens for different purposes.

The shaft 20 is connected to the connector 8 on said proximal end 24.

In particular, the shaft 20 is connected, at its proximal end 24, to the connector 8 so that said guide wire lumen 32 and inflation lumen 36 are mechanically and fluidically connected with said guide wire channel 12 and inflation channel 16 of the connector 8, respectively.

According to an embodiment of the present invention, the shaft 20 and the connector 8 are made up of a polymer material, such as Polyamide, Pebax, Polycarbonate and similar.

According to the invention, the shaft is provided with an inflatable balloon 48, which is fluidically connected with said inflation lumen 36 in order to be selectively inflated and/or deflated.

According to one example, the balloon is provided to a couple of markers 40, positioned in correspondence of two ends of the balloon 48, defining the axial length of the balloon itself.

Advantageously, the balloon 48 is covered with a drug to be delivered on a target lesion.

The drug can be of any type, depending on the kind and positioning of lesion to be treated.

According to one example, the drug formulation is a Paclitaxel solution.

According to the invention, an external wall 52 of the shaft 20, opposite to said lumens 32,36, is covered with a lubricant.

In this way, said external wall 52 which, during insertion of the catheter into the vessel, contacts the internal wall of the blood vessel or the internal wall of an introducer, sheath, guiding catheter or any other accessory used for intervention, is lubricated and can easily slip into the vessel itself.

According to the invention, the external wall 52 of the shaft 20 is covered with a lubricant except for said inflatable balloon 48.

In this way, the lubricant does not interfere with the action of the drug which has to be delivered on the target lesion.

The distal end 28 of the shaft 20 comprises a flexible tip 56 which aims the catheter to be cannulated inside blood vessels.

According to one embodiment, said tip 56 is covered with lubricant too.

Advantageously, said guidewire lumen 32 is internally covered with a lubricant.

Therefore, the shaft is provided with lubricant coating both externally, on the external wall 52, and internally, on the guidewire lumen 32.

According to one embodiment, said lubricant is a hydrophobic lubricant, like silicon or a hydrophilic lubricant.

According to a possible embodiment, the lubricant applied to the external wall 52 is a hydrophobic lubricant only, while the lubricant applied on the guidewire lumen 32 can be both hydrophilic and hydrophobic.

Hereafter the method of manufacturing a catheter according to the present invention will be described.

In particular the method of producing a catheter 4 according the invention comprises the steps of providing a shaft 20 extending from a proximal end 24 to a distal end 28 along an axial direction X-X, the shaft 20 having a guide wire lumen 32 and an inflation lumen 36.

The shaft 20 is provided with an inflatable balloon 48, fluidically connected with said inflation lumen 36 in order to be selectively inflated and/or deflated.

The method comprises the steps of:
- coating the inflatable balloon 48 with a drug to be delivered on a target lesion,
- coating with a lubricant the external wall 52 of the shaft 20, opposite to said lumens 32,36,
- applying a lubricant into said guidewire lumen 32, in order to internally cover with a lubricant the guidewire lumen 32.

In particular, according to a possible and not limitative embodiment, the phase of applying lubricant into said guidewire lumen 36 comprises the steps of:
- flushing, spraying or injecting the guidewire lumen 36 with a lubricating solution,
- flushing the guidewire lumen 36 with compressed air to release coating in excess.

According to other possible embodiments, lubricant may be applied by plasma deposition.

According to an embodiment, the phase of applying lubricant into said guidewire lumen 32 comprises the steps of:
- flushing the guidewire lumen 32 with injection of a lubricant coating solution,
- coating only a part of the shaft 20 comprised between the balloon 48 and the connector 8.

Moreover, the phase of applying lubricant to the shaft 20 comprises the step of coating the external wall 52 of the shaft 20 by dipping it into a lubricant solution or by flushing or spraying it with a lubricant solution without influencing the balloon 48; also plasma deposition may be used in order to treat to the external wall 52 of the shaft 20.

As it can be seen from the description, the catheter and relative method of production according to the invention makes it possible to overcome the drawbacks mentioned with reference to the prior art.

In particular, it is possible to provide a DCB catheter which can reach the target lesion easily, without jamming and without damaging the cannulated vessel.

Therefore the use of the DCB catheter according to the present invention is advantageous because it is possible to reach the target lesion with lower friction and therefore less push, minimizing risk to damage the internal wall of the cannulated vessel also in the case of bifurcations or severe bending of the vessel itself.

Moreover, the DCB catheter may easily run along its guidewire without jamming: this is very useful for the user because he can 'feel' the geometry of the cannulated vessel, while pushing the DCB catheter throughout the vessel, without being confused by internal resistance of the catheter against the guidewire lumen. In this way the user can feel the geometry of the vessel and then can push forward or pull rearward the catheter basing on the resistance due to the anatomical geometry only, without being misled by internal friction and without risking to damage the internal wall of the cannulated vessel.

Of course the lubricant used both internally and externally with respect to the catheter does not alter the functionality and the active principle of the drug of the balloon.

Moreover, it is possible to use DCB catheters, which have bigger and longer balloons than other types of balloon catheters, in order to treat any type of lesion or blood vessel, since the higher friction encountered by said DCB catheters is compensated and overcome by a more efficient lubrication of the catheter itself.

Therefore the risk of jamming or grasping within blood vessel is really decreased when using a DCB catheter according to the present invention.

Moreover, the use of lubricant coating according to the present invention is particular advantageous with a catheter having a dual lumen construction wherein said lumens are not coaxial each other. In fact, such a dual lumen geometry cannot be obtained by a multilayer extrusion with an inner material layer with lower friction than an outer material layer. Therefore, the use of a lubricant coating helps reducing friction significantly in a dual layer, not coaxial, catheter.

A person skilled in the art may make numerous modifications and variations to the catheters and methods described above so as to satisfy contingent and specific requirements, while remaining within the scope of protection of the invention as defined by the following claims.

## Claims

1. A drug coated balloon (DCB) catheter (4) comprising
- a connector (8),
- a shaft (20) extending from a proximal end (24) to a distal end (28) along an axial direction (X-X) and having a guidewire lumen (32) and an inflation lumen (36), the shaft being connected to the connector (8) on said proximal end (24),
- the catheter (4) having at least a dual-lumen construction provided with at least two lumens comprising said guidewire lumen (32) and said inflation lumen (36), wherein said lumens (32,36) are separated each other by an internal sept (44),
- the shaft (20) being provided with an inflatable balloon (48), fluidically connected with said inflation lumen (36) in order to be selectively inflated and/or deflated,
**characterised in that**
- said internal sept (44) delimits, at least partly, a wall of each of the separated lumens (32,36),
- the balloon (48) is covered with a drug to be delivered on a target lesion,
- said guidewire lumen (32) is internally covered with a lubricant,
wherein an external wall (52) of the shaft (20), opposite to said lumens (32,36), is covered with a lubricant.

2. Drug coated balloon (DCB) catheter (4) according to claim 1, wherein the entire external wall (52) of the shaft (20) is covered with a lubricant except for said balloon (48).

3. Drug coated balloon (DCB) catheter (4) according to any one of claims 1 to 2, wherein the distal end (28) of the shaft (20) comprises a flexible tip (56), said tip (56) being covered with lubricant.

4. Drug coated balloon (DCB) catheter (4) according to any of claims 1 to 3, wherein said lubricant is a hydrophilic lubricant.

5. Drug coated balloon (DCB) catheter (4) according to any one of claims 1 to 3, wherein said lubricant is a hydrophobic lubricant like silicon.

6. Drug coated balloon (DCB) catheter (4) according to any one of claims 1 to 3, wherein the lubricant applied to an external wall (52) of the shaft (20) is a hydrophobic lubricant, and the lubricant applied on the guidewire lumen (32) is hydrophilic or hydrophobic.

7. Drug coated balloon (DCB) catheter (4) according to any one of the previous claims, wherein said connector (8) comprises at least a guide wire channel (12) and an inflation channel (16),
- the shaft (20) being connected, at its proximal end (24), to the connector (8) so that said guide wire lumen (32) and inflation lumen (36) are mechanically and fluidically connected with said guide wire channel (12) and inflation channel (16) respectively.

8. Drug coated balloon (DCB) catheter (4) according to any one of the previous claims, wherein the shaft (20) and the connector (8) are made up of a polymer material.

9. Method of producing a drug coated balloon catheter (4) according to any one of preceding claims, comprising the steps of:
- providing a shaft (20) extending from a proximal end (24) to a distal end (28) along an axial direction (X-X), the shaft (20) having a guide wire lumen (32) and an inflation lumen (36),
- the shaft (20) being provided with an inflatable balloon (48), fluidically connected with said inflation lumen (36) in order to be selectively inflated,
- the catheter (4) having at least a dual-lumen construction provided with at least two lumens comprising said guidewire lumen (32) and said inflation lumen (36), wherein said lumens (32,36) are separated each other by an internal sept (44),
**characterised in that** it comprises the steps of:
- coating the balloon (48) with a drug to be delivered on a target lesion,
- coating with a lubricant an external wall (52) of the shaft (20), opposite to said lumens (32,36),
- applying a lubricant into said guidewire lumen (32), in order to internally cover with a lubricant the guidewire lumen (32),
- wherein said internal sept (44) delimits, at least partly, a wall of each of the separated lumens (32,36).

10. Method of producing a drug coated balloon catheter (4) according to claim 9, wherein the phase of applying lubricant into said guidewire lumen (32) comprises the steps of:
- flushing, spraying or injecting the guidewire lumen (32) with a lubricating solution, or using plasma deposition,
- flushing the guidewire lumen (32) with compressed air to release coating in excess.

11. Method of producing a drug coated balloon catheter (4) according to claim 9 or 10, wherein the phase of applying lubricant into said guidewire lumen (32) comprises the steps of:
- flushing the guidewire lumen (32) with injection of a lubricant coating solution,
- coating only a part of the shaft (20) comprised between the balloon (48) and the connector (8).

12. Method of producing a drug coated balloon catheter (4) according to claim 9, 10 or 11, wherein the phase of applying lubricant to the shaft (20) comprises the steps of:
- coating the external wall (52) of the shaft (20) by dipping it into a lubricant solution or by spraying or flushing it with a lubricant solution without influencing the drug coated balloon (48).

## Patentansprüche

1. Medikamentenbeschichteter Ballon(DCB)katheter (4), umfassend
- einen Verbinder (8),
- einen Schaft (20), der sich von einem proximalen Ende (24) zu einem distalen Ende (28) entlang einer axialen Richtung (X-X) erstreckt und ein Führungsdrahtlumen (32) und ein Aufblaslumen (36) aufweist, wobei der Schaft mit dem Verbinder (8) an dem proximalen Ende (24) verbunden ist,
- wobei der Katheter (4) zumindest eine Dual-Lumen-Konstruktion aufweist, die mit zumindest zwei Lumen versehen ist, die das Führungsdrahtlumen (32) und das Aufblaslumen (36) umfassen, wobei die Lumen (32, 36) durch ein inneres Septum (44) voneinander getrennt sind,
- wobei der Schaft (20) mit einem aufblasbaren Ballon (48) versehen ist, der mit dem Aufblaslumen (36) fluidisch verbunden ist, um selektiv aufgeblasen und/oder entleert zu werden,
**dadurch gekennzeichnet**
- das innere Septum (44) zumindest teilweise eine Wand jedes der getrennten Lumen (32, 36) begrenzt,
- der Ballon (48) mit einem Medikament bedeckt ist, das auf eine Zielläsion abzugeben ist,
- das Führungsdrahtlumen (32) innen mit einem Gleitmittel bedeckt ist,
wobei eine Außenwand (52) des Schafts (20), die dem Lumen (32, 36) gegenüberliegt bzw. entgegengesetzt ist, mit einem Gleitmittel bedeckt ist.

2. Medikamentenbeschichteter Ballon(DCB)katheter (4) nach Anspruch 1, wobei die gesamte Außenwand (52) des Schafts (20) mit Ausnahme des Ballons (48) mit einem Gleitmittel bedeckt ist.

3. Medikamentenbeschichteter Ballon(DCB)katheter (4) nach einem der Ansprüche 1 bis 2, wobei das distale Ende (28) des Schafts (20) eine flexible Spitze (56) umfasst, wobei die Spitze (56) mit Gleitmittel bedeckt ist.

4. Medikamentenbeschichteter Ballon(DCB)katheter (4) nach einem der Ansprüche 1 bis 3, wobei das Gleitmittel ein hydrophiles Gleitmittel ist.

5. Medikamentenbeschichteter Ballon(DCB)katheter (4) nach einem der Ansprüche 1 bis 3, wobei das Gleitmittel ein hydrophobes Gleitmittel wie Silikon ist.

6. Medikamentenbeschichteter Ballon(DCB)katheter (4) nach einem der Ansprüche 1 bis 3, wobei das auf eine Außenwand (52) des Schafts (20) aufgebrachte Gleitmittel ein hydrophobes Gleitmittel ist und das Gleitmittel auf das Führungsdrahtlumen (32) aufgebrachte Gleitmittel hydrophil oder hydrophob ist.

7. Medikamentenbeschichteter Ballon(DCB)katheter (4) nach einem der vorhergehenden Ansprüche, wobei der Verbinder (8) zumindest einen Führungsdrahtkanal (12) und einen Aufblaskanal (16) umfasst,
- wobei der Schaft (20) an seinem proximalen Ende (24) mit dem Verbinder (8) verbunden ist, so dass das Führungsdrahtlumen (32) und das Aufblaslumen (36) mechanisch und fluidisch mit dem Führungsdrahtkanal (12) bzw. dem Aufblaskanal (16) verbunden sind.

8. Medikamentenbeschichteter Ballon(DCB)katheter (4) nach einem der vorhergehenden Ansprüche, wobei der Schaft (20) und der Verbinder (8) aus einem Polymermaterial bestehen.

9. Verfahren zum Herstellen eines medikamentenbeschichteten Ballonkatheters (4) nach einem der vorhergehenden Ansprüche, umfassend die Schritte:
- Bereitstellen eines Schafts (20), der sich von einem proximalen Ende (24) zu einem distalen Ende (28) entlang einer axialen Richtung (X-X) erstreckt, wobei der Schaft (20) ein Führungsdrahtlumen (32) und ein Aufblaslumen (36) aufweist,
- wobei der Schaft (20) mit einem aufblasbaren Ballon (48) versehen ist, der mit dem Aufblaslumen (36) fluidisch verbunden ist, um selektiv aufgeblasen zu werden,
- wobei der Katheter (4) zumindest eine Dual-Lumen-Konstruktion aufweist, die mit zumindest zwei Lumen versehen ist, die das Führungsdrahtlumen (32) und das Aufblaslumen (36) umfassen, wobei die Lumen (32, 36) durch ein inneres Septum (44) voneinander getrennt sind,
**dadurch gekennzeichnet, dass** es die Schritte umfasst:
- Beschichten des Ballons (48) mit einem Medikament, das auf eine Zielläsion abzugeben ist,
- Beschichten einer Außenwand (52) des Schafts (20), die den Lumen (32, 36) gegenüberliegt bzw. entgegengesetzt ist, mit einem Gleitmittel,
- Aufbringen eines Gleitmittels in das Führungsdrahtlumen (32), um das Führungsdrahtlumen (32) innen mit einem Glietmittel zu bedecken,
- wobei das innere Septum (44) zumindest teilweise eine Wand jedes der getrennten Lumen (32, 36) begrenzt.

10. Verfahren zum Herstellen eines medikamentenbeschichteten Ballonkatheters (4) nach Anspruch 10, wobei die Phase des Aufbringens von Gleitmittel in das Führungsdrahtlumen (32) die Schritte umfasst:
- Spülen, Besprühen oder Injizieren des Führungsdrahtlumens (32) mit einer Gleitlösung oder Verwendung von Plasmaabscheidung,
- Spülen des Führungsdrahtlumens (32) mit Druckluft, um überschüssige Beschichtung zu entfernen.

11. Verfahren zum Herstellen eines medikamentenbeschichteten Ballonkatheters (4) nach Anspruch 9 oder 10, wobei die Phase des Aufbringens von Gleitmittel in das Führungsdrahtlumen (32) die Schritte umfasst:
- Spülen des Führungsdrahtlumens (32) mit Injektion einer Gleitbeschichtungslösung,
- Beschichten nur eines Teils des Schafts (20), der zwischen dem Ballon (48) und dem Verbinder (8) enthalten ist.

12. Verfahren zum Herstellen eines medikamentenbeschichteten Ballonkatheters (4) nach Anspruch 9, 10 oder 11, wobei die Phase des Aufbringens von Gleitmittel auf den Schaft (20) die Schritte umfasst:
- Beschichten der Außenwand (52) des Schafts (20) durch Eintauchen derselben in eine Gleitmittellösung oder durch Besprühen oder Spülen derselben mit einer Gleitmittellösung ohne Beeinträchtigung des medikamentenbeschichteten Ballons (48).

## Revendications

1. Cathéter (4) à ballonnet revêtu de médicament (DCB) comprenant
- un connecteur (8),
- une tige (20) s'étendant d'une extrémité proximale (24) à une extrémité distale (28) le long d'une direction axiale (X-X) et ayant une lumière de fil-guide (32) et une lumière de gonflage (36), la tige étant reliée au connecteur (8) sur ladite extrémité proximale (24),
- le cathéter (4) ayant au moins une construction à double lumière pourvue d'au moins deux lumières comprenant ladite lumière de fil-guide (32) et ladite lumière de gonflage (36), lesdites lumières (32, 36) étant séparées l'une de l'autre par un septum interne (44),
- la tige (20) étant pourvue d'un ballonnet gonflable (48), relié de manière fluidique à ladite lumière de gonflage (36) afin d'être sélectivement gonflé et/ou dégonflé,
**caractérisé en ce que**
- ledit septum interne (44) délimite, au moins partiellement, une paroi de chacune des lumières séparées (32, 36),
- le ballonnet (48) est recouvert d'un médicament à délivrer sur une lésion cible,
- ladite lumière de fil-guide (32) est recouverte de manière interne d'un lubrifiant,
une paroi externe (52) de la tige (20), opposée auxdites lumières (32, 36), étant recouverte d'un lubrifiant.

2. Cathéter (4) à ballonnet revêtu de médicament (DCB) selon la revendication 1, toute la paroi externe (52) de la tige (20) étant recouverte d'un lubrifiant à l'exception dudit ballonnet (48).

3. Cathéter (4) à ballonnet revêtu de médicament (DCB) selon l'une quelconque des revendications 1 à 2, l'extrémité distale (28) de la tige (20) comprenant une pointe flexible (56), ladite pointe (56) étant recouverte de lubrifiant.

4. Cathéter (4) à ballonnet revêtu de médicament (DCB) selon l'une quelconque des revendications 1 à 3, ledit lubrifiant étant un lubrifiant hydrophile.

5. Cathéter (4) à ballonnet revêtu de médicament (DCB) selon l'une quelconque des revendications 1 à 3, ledit lubrifiant étant un lubrifiant hydrophobe de type silicone.

6. Cathéter (4) à ballonnet revêtu de médicament (DCB) selon l'une quelconque des revendications 1 à 3, le lubrifiant appliqué sur une paroi externe (52) de la tige (20) étant un lubrifiant hydrophobe, et le lubrifiant appliqué sur la lumière de fil-guide (32) étant hydrophile ou hydrophobe.

7. Cathéter (4) à ballonnet revêtu de médicament (DCB) selon l'une quelconque des revendications précédentes, ledit connecteur (8) comprenant au moins un canal de fil-guide (12) et un canal de gonflage (16),
- la tige (20) étant reliée, à son extrémité proximale (24), au connecteur (8) de sorte que lesdites lumière de fil-guide (32) et lumière de gonflage (36) sont reliées mécaniquement et fluidiquement auxdits canal de fil-guide (12) et canal de gonflage (16), respectivement.

8. Cathéter (4) à ballonnet revêtu de médicament (DCB) selon l'une quelconque des revendications précédentes, la tige (20) et le connecteur (8) étant constitués d'un matériau polymère.

9. Procédé de production d'un cathéter (4) à ballonnet revêtu de médicament selon l'une quelconque des revendications précédentes, comprenant les étapes consistant à :
- fournir une tige (20) s'étendant d'une extrémité proximale (24) à une extrémité distale (28) le long d'une direction axiale (X-X), la tige (20) ayant une lumière de fil-guide (32) et une lumière de gonflage (36),
- la tige (20) étant pourvue d'un ballonnet gonflable (48), relié de manière fluidique à ladite lumière de gonflage (36) afin d'être sélectivement gonflé,
- le cathéter (4) ayant au moins une construction à double lumière pourvue d'au moins deux lumières comprenant ladite lumière de fil-guide (32) et ladite lumière de gonflage (36), lesdites lumières (32, 36) étant séparées l'une de l'autre par un septum interne (44),
**caractérisé en ce qu'**il comprend les étapes consistant à :
- revêtir le ballonnet (48) d'un médicament à délivrer sur une lésion cible,
- revêtir d'un lubrifiant une paroi externe (52) de la tige (20), opposée auxdites lumières (32, 36),
- appliquer un lubrifiant dans ladite lumière de fil-guide (32), afin de recouvrir la lumière de fil-guide (32) de manière interne à l'aide d'un lubrifiant,
- ledit septum interne (44) délimitant, au moins partiellement, une paroi de chacune des lumières séparées (32, 36).

10. Procédé de production d'un cathéter (4) à ballonnet revêtu de médicament selon la revendication 9,
la phase d'application d'un lubrifiant dans ladite lumière de fil-guide (32) comprenant les étapes consistant à :
- rincer, pulvériser ou injecter la lumière de fil-guide (32) à l'aide d'une solution lubrifiante, ou utiliser un dépôt de plasma,
- rincer la lumière de fil-guide (32) à l'aide d'air comprimé pour éliminer un revêtement en excès.

11. Procédé de production d'un cathéter (4) à ballonnet revêtu de médicament selon la revendication 9 ou 10, la phase d'application d'un lubrifiant dans ladite lumière de fil-guide (32) comprenant les étapes consistant à :
- rincer la lumière de fil-guide (32) à l'aide d'une injection d'une solution de revêtement lubrifiante,
- revêtir seulement une partie de la tige (20) comprise entre le ballonnet (48) et le connecteur (8).

12. Procédé de production d'un cathéter (4) à ballonnet revêtu de médicament selon la revendication 9, 10 ou 11, la phase d'application d'un lubrifiant sur la tige (20) comprenant l'étape consistant à :
- revêtir la paroi externe (52) de la tige (20) en la trempant dans une solution lubrifiante ou en la pulvérisant ou en la rinçant à l'aide d'une solution lubrifiante sans influencer le ballonnet revêtu de médicament (48).
